# EUROPÄISCHE PATENTSCHRIFT

(11) **EP 2 590 587 B1**
(45) Veröffentlichungstag und Bekanntmachung des Hinweises auf die Patenterteilung: **15.07.2015**
(21) Anmeldenummer: 11725750.1
(22) Anmeldetag: 16.06.2011
(51) Int. Cl.: A61B 19/00, B25J 15/00

(54) **ROBOTERSTRUKTUR**
ROBOT STRUCTURE
STRUCTURE DE ROBOT

(30) Priorität: 06.07.2010 DE 102010026305
(43) Veröffentlichungstag der Anmeldung: 15.05.2013
(73) Patentinhaber: Deutsches Zentrum für Luft- und Raumfahrt e.V., 51147 Köln (DE)
(72) Erfinder: SEIBOLD, Ulrich, Burnaby, British Columbia V3N 4Z4 (CA); KÜBLER, Bernhard, 74229 Oedheim (DE); LANTERMANN, Sophie, 81679 München (DE); HAGN, Ulrich, 82396 Pähl (DE)
(74) Vertreter: Von Kreisler Selting Werner - Partnerschaft von Patentanwälten und Rechtsanwälten mbB
(86) Internationale Anmeldenummer: PCT/EP2011/060054
(87) Internationale Veröffentlichungsnummer: WO 2012/004109

(56) Entgegenhaltungen:
- WO-A1-00/51486
- DE-A1-102007 022 122
- DE-B3-102006 059 952

## Beschreibung

Die Erfindung betrifft eine Roboterstruktur, insbesondere für die minimal-invasive Chirurgie.

In der robotergestützten minimal-invasiven Chirurgie werden Instrumente wie Greif- oder Schneidinstrumente an einem distalen Ende einer Roboterstruktur vorgesehen. Die Instrumente befinden sich während der Operation im Körperinneren des Patienten. Um die mit der Roboterstruktur verbundenen Instrumente betätigen und bewegen zu können, weist die Roboterstruktur mindestens zwei Roboterelemente auf, die über ein Gelenk oder mehrere Gelenke miteinander verbunden sind. Hierbei ist ein erstes Roboterelement beispielsweise als Roboterarm und das zweite, bewegbare Roboterelement als Greif- oder Scherenmechanismus ausgebildet. Zum Betätigen des bewegbaren Roboterelementes ist mit diesem eine Kraftübertragungseinrichtung verbunden.

Zur Messung der auftretenden Kräfte, insbesondere der Kontakt- und Greifkräfte, ist mit der Roboterstruktur ein Sensorelement verbunden. Grundsätzlich besteht dabei die Problematik, dass die Messung der Kontaktkräfte und Greifkräfte des bewegbaren Roboterelements nur mit sehr schlechter Auflösung gemessen werden können, da die auftretenden Kräfte von erheblich großen Antriebs- bzw. Bewegungskräften von der Kraftübertragungseinrichtung auf das bewegbare Roboterelement überlagert werden. Hierbei sind Greifkräfte diejenigen Kräfte, die beim Greifen eines Objektes entstehen, das nicht mit der Umgebung in Kontakt steht. Kontaktkräfte entstehen durch die Interaktion eines Instruments oder eines gegriffenen Objekts mit der Umgebung. Hierbei sind die Kontaktkräfte unabhängig von dem Greifzustand. Die Kontaktkräfte weisen sechs Freiheitsgrade, nämlich drei Kräfte und drei Momente auf.

Zum Messen der Greifkraft ist es beispielsweise bekannt, die Antriebs- bzw. Bewegungskraft zu messen. Die Antriebskraft ist hierbei ein Maß für die Greifkraft und hat daher keine störenden Einflüsse. Die Messung der Greifkraft ist jedoch relativ ungenau. Ein derartiges Instrument ist in J. Rosen, B. Hannaford, M. MacFarlane und M. Sinanan, "Force Controlled and Teleoperated Endoscopic Grasper for minimally Invasive Surgery - Experimental Performance Evaluation", IEEE Transactions on Biomedical Engineering, 1999 und G. Tholey, A. Pillarisetti, W. Green und J. Desai, "Design, Development and Testing of an Automated Laparoscopic Grasper with 3D Force Measurement Capability", Medical Simulation: International Symposium, ISMS 2004, beschrieben.

Ferner ist es zum Messen der auftretenden Kräfte bekannt, in den Greifbacken entsprechende Sensoren, beispielsweise Drucksensoren, vorzusehen. Dieses in G. Tholey, A. Pillarisetti, W. Green und J. Desai, "Design, Development, and Testing of an Automated Laparoscopic Grasper with 3D Force Measurement Capability", Medical Simulation: International Symposium, ISMS 2004 beschriebene Instrument weist jedoch den Nachteil auf, dass für die Integration von Sensoren nur ein geringes Bauvolumen vorhanden ist, da die Greifbacken insbesondere in der minimal-invasiven Chirurgie möglichst klein ausgebildet sein müssen.

Ferner ist in H. Mayr, I. Nagy, A. Knoll, E. Schirmbeck und R. Bauernschmitt, "Upgrading Instruments for Robotic Surgery", Australasian Conference on Robotics & Automation, 2004, ein Instrument beschrieben, bei dem Bowdenzüge zur Übertragung der Antriebskräfte mit Hilfe eines Zugmediums vorgesehen sind. Die Abstützung der Kräfte erfolgt hierbei über einen rigiden Mantel, der biegeweich aber in axialer Richtung zugsteif ausgebildet ist. Der Mantel dient hierbei sowohl zum Schließen des Kraftflusses der Antriebskräfte, bei denen es sich um relativ hohe Kräfte handelt, als auch zum Messen der Kontaktkräfte, die vergleichsweise gering sind. Die Bowdenzüge stützen zumindest einen Teil der zu messenden Axialkräfte ab.

Dies hat eine Verschlechterung der Messergebnisse zur Folge. Der Einfluss der Abstützung ist hierbei von der Lage der Bowdenzüge relativ zum Sensor abhängig. Die Beeinflussung der Abstützung kann daher variieren und ist mathematisch nicht oder nur ungenügend zu kompensieren.

Selbst bei geschickter Konstruktion überlagern die durch die Bowdenzüge übertragenen Antriebskräfte zumindest teilweise die Kontaktkräfte. Der Effekt ist unter anderem dadurch bedingt, dass sich der Mantel des Bowdenzuges beim Auftreten axialer Zug- oder Druckkräfte zwangsläufig dehnt oder verkürzt, wodurch die Einleitung unerwünschter Antriebskräfte unvermeidlich ist.

Aus der DE 10 2006 059 952 der Anmelderin ist eine Roboterstruktur bekannt, bei der Greifelemente eines Roboterelementes über Seilzüge angetrieben werden. Die Antriebskräfte können dabei an der Basis des Sensors abgestützt werden und gehen somit nicht in das Messergebnis ein.

Eine Lösung, basierend auf Seilzügen, geht jedoch von der weitestgehend reibungs- und verlustfreien Übertragung von Kräften und einer entsprechenden Führung der Seile aus. Aufgrund von Fertigungstoleranzen und Materialeigenschaften sind diese Voraussetzungen jedoch bei einer starken Miniaturisierung des Instruments, wie es beispielsweise für die minimalinvasive Chirurgie wünschenswert ist, nicht oder nur mit großem Aufwand einzuhalten. Dadurch ist die Fertigung und Montage der Mechanik derartiger Instrumente sehr aufwändig und kostspielig. Ferner sind Seilzüge als Antriebselemente nur bedingt sterilisierbar, sodass auch der Einsatz derartiger Instrumente in der minimalinvasiven Chirurgie begrenzt ist.

Aus der DE 10 2007 022 122 A1 ist eine Chirurgie-Roboteranordnung bekannt, bei der die Antriebskraft ebenfalls an der Basis des Sensors abgestützt werden kann, wobei die Roboteranordnung ein hydraulisches Antriebsprinzip besitzt. In der minimalinvasiven Chirurgie ist jedoch die Verwendung von hydraulischen Komponenten häufig nicht erwünscht.

Es ist daher die Aufgabe der vorliegenden Erfindung, eine Roboterstruktur bereitzustellen, mit der die Greif- und Kontaktkräfte der Greif- oder Schneidinstrumente mit einer hohen Genauigkeit messbar sind und die Nachteile des Standes der Technik vermieden werden. Dabei sollen möglichst eine weitere Miniaturisierung der Roboterstruktur und eine gute Sterilisierbarkeit der Antriebselemente realisierbar sein.

Zur Lösung der Aufgabe dienen die Merkmale des Anspruchs 1.

Erfindungsgemäß ist eine Roboterstruktur, insbesondere für die minimalinvasive Chirurgie mit einem ersten Roboterelement und einem dazu relativ bewegbaren zweiten Roboterelement vorgesehen, welches zwei gleichmäßig gegeneinander bewegbare Greif- und/oder Schneidelemente aufweist, die über eine Gelenkachse mit dem ersten Roboterelement verbunden sind. Die Roboterstruktur weist ferner eine Übertragungseinrichtung zum Bewegen der Greif- und/oder Schneidelemente des bewegbaren Roboterelements und mindestens ein Sensorelement mit einem sensitiven Ende zur Aufnahme von einem bewegbaren Roboterelement auftretenden Kräften und/oder Momenten und mit einem fest mit dem ersten Roboterelement verbundenen Basiselement auf. Die Gelenkachse ist zur Aufnahme der durch Greif- und/oder Kontaktkräfte an dem Greif- und/oder Schneidelementen verursachten Kräfte und/oder Momente in einem Trägerelement gelagert und das Trägerelement ist mit dem sensitiven Ende jedes Sensorelementes verbunden. Die Kraftübertragungseinrichtung ist jeweils über ein Antriebselement mit einem der Greif- und/oder Schneidelemente zum Einleiten der Bewegung verbunden, wobei die Antriebselement einander entgegengerichtet und orthogonal oder zumindest weitestgehend orthogonal zu der Mittelachse der Roboterstruktur angeordnet sind und während des gesamten Bewegungsvorgangs der Greif- und/oder Schneidelemente in der orthogonalen Anordnung zu der Mittelachse verbleiben.

Durch die Anordnung der Antriebselemente orthogonal zu der Mittelachse der Roboterstruktur und einander entgegengerichtet, werden die von den Antriebselementen auf die Greif- und/der Schneidelemente ausgeübten Kräfte ausschließlich oder zumindest weitestgehend ebenfalls in einer Richtung orthogonal zu der Mittelachse in die Greif- und/oder Schneidelemente eingeleitet. Die Antriebskräfte überlagern sich an der Gelenkachse und in dem Trägerelement, wobei aufgrund der einander entgegengerichteten Anordnung der Antriebselemente auch die Antriebskräfte an der Gelenkachse einander entgegengerichtet sind und sich somit zumindest weitestgehend aufheben. Dadurch wird verhindert oder zumindest weitestgehend verhindert, dass Kraftanteile, die von den Antriebselementen auf die Greif- und/oder Schneidelemente ausgeübt werden, über das Trägerelement auf die sensitiven Enden der Sensorelemente übertragen werden. Dadurch geht die Antriebskraft nicht mit in das von den Sensorelementen gemessene Messergebnis ein, sodass eine sehr genaue Bestimmung der Greif- und/oder Kontaktkräfte möglich ist.

Die Antriebselemente können beispielsweise als Stange ausgebildet sein. Über eine Stange ist in vorteilhafter Weise eine Kraftübertragung auf die Greif- und/oder Schneidelemente möglich, wobei die Stange auf konstruktiv einfache Art und Weise in der orthogonalen Anordnung zu der Mittelachse der Roboterstruktur gehalten werden kann. Über als Stangen ausgebildete Antriebselemente können in vorteilhafter Weise Zug- und Druckkräfte in einer Richtung orthogonal zu der Mittelachse der Roboterstruktur in die Greif- und/oder Schneidelemente eingebracht werden, die einander entgegengerichtet sind, sodass die Kraftkomponenten sich in der Gelenkachse aufheben.

Die Kraftübertragungseinrichtung kann beispielsweise Führungselemente aufweisen, wobei jeweils ein Führungselement ein Antriebselement führt, derart, dass jedes Antriebselement während des gesamten Bewegungsvorgangs der Greif- und/der Schneidelemente in der orthogonalen Anordnung zu der Mittelachse verbleiben.

Dabei können die Führungselemente beispielsweise als Führungshebel ausgebildet sein. Auf diese Weise wird auf eine konstruktiv einfache Art und Weise gewährleistet, dass die Antriebselemente stets in der orthogonalen Anordnung zu der Mittelachse der Roboterstruktur angeordnet sind.

Es kann vorgesehen sein, dass die Führungselemente jeweils mit dem ersten Roboterelement und/oder mit dem Basiselement eines der Sensorelemente verbunden sind, vorzugsweise über ein Verbindungselement. Mit einer derartigen Konstruktion wird ermöglicht, dass von der Kraftübertragungseinrichtung auf die Führungselemente ausgeübte Axialkräfte über die Verbindungselemente auf das erste Roboterelement und/oder auf die Basiselemente der Sensorelemente übertragen werden. Dadurch wird verhindert, dass die Axialkräfte über die Antriebselemente, die Greif- und/oder Schneidelemente und die Gelenkachse auf das Trägerelement und somit auf die sensitiven Enden der Sensorelemente übertragen werden. Axiale Kräfte der Kraftübertragungseinrichtung gehen somit nicht in das Messergebnis ein, sodass eine sehr genaue Messung der Greif- und/oder Kontaktkräfte ermöglicht wird.

Nach einem Ausführungsbeispiel der Erfindung ist vorgesehen, dass die Kraftübertragungseinrichtung für jedes Antriebselement eine Kulissenführung aufweist, die jeweils ein Antriebselement antreibt. Dabei weist jedes Antriebselement vorzugsweise einen Stift auf, der in der Kulissenführung geführt ist. Eine derartige Anordnung ist auf konstruktiv einfache Art und Weise realisierbar, wobei aufgrund der einfachen mechanischen Ausgestaltung eine gute Sterilisierbarkeit gegeben ist.

Die Kulissenführungen können jeweils eine Führungsnut aufweisen, die in einem Winkel zu der Mittelachse der Roboterstruktur angeordnet ist. Eine derartige Führungsnut ermöglicht eine Übertragung der Antriebskraft von der Kraftübertragungseinrichtung auf die Führungselemente und die Antriebselemente in vorteilhafter Weise, wobei über eine Bewegung der Kraftübertragungseinrichtung in axialer Richtung der Roboterstruktur eine Kraft in eine Richtung orthogonal zu der Mittelachse der Roboterstruktur in den Antriebselementen erzeugbar ist. Dadurch kann durch ein einfaches Bewegen der Kraftübertragungseinrichtung in axiale Richtung der Roboterstruktur die gewünschte Antriebskraft in den Antriebselementen erzeugt werden.

In einem alternativen Ausführungsbeispiel der Erfindung treibt die Kraftübertragungseinrichtung die Antriebselemente über Hebel an.

Es kann vorgesehen sein, dass die Kraftübertragungseinrichtung über eine Zugdruckstange antreibbar ist. In der Abhängigkeit von der konstruktiven Ausgestaltung der erfindungsgemäßen Roboterstruktur ist über eine Zug- oder Druckbewegung der Zug/Druckstange und somit der Kraftübertragungseinrichtung ein entsprechendes Öffnen oder Schließen der Greif- und/oder Schneidelemente möglich.

Alternativ kann die Kraftübertragungseinrichtung über eine Zugstange oder über eine Druckstange antreibbar sein, wobei die Zugstange oder die Druckstange vorzugsweise federvorgespannt ist. Durch die Federvorspannung wird gewährleistet, dass nach dem Einleiten einer Bewegung an die Kraftübertragungseinrichtung beispielsweise ein Schließen der Greif- und/oder Schneidelemente diese Bewegung entgegen der Federvorspannung erfolgt und über die Federvorspannung die Greif- und/oder Schneidelemente wieder in die geöffnete Position zurückbewegt werden.

In einem Ausführungsbeispiel der Erfindung kann auch vorgesehen sein, dass die Kraftübertragungseinrichtung über ein Zugseil antreibbar ist, wobei das Zugseil vorzugsweise federvorgespannt ist.

Im Folgenden wird die Erfindung unter Bezugnahme auf die nachfolgenden Figuren näher erläutert. Es zeigen:
- Fig.1: eine schematische Seitenansicht einer erfindungsgemäßen Roboterstruktur mit geschlossenen Greif- und/oder Schneidelementen,
- Fig. 2: eine schematische Seitenansicht der Roboterstruktur der Fig. 1 mit geöffnetem Greif- und/oder Schneidelementen,
- Fig. 3: eine schematische Seitenansicht eines zweiten Ausführungsbeispiels einer erfindungsgemäßen Roboterstruktur mit geschlossenem Greif- und/oder Schneidelement und
- Fig. 4: eine schematische Seitenansicht der Roboterstruktur der Fig. 3 mit geöffnetem Greif- und/oder Schneidelement.

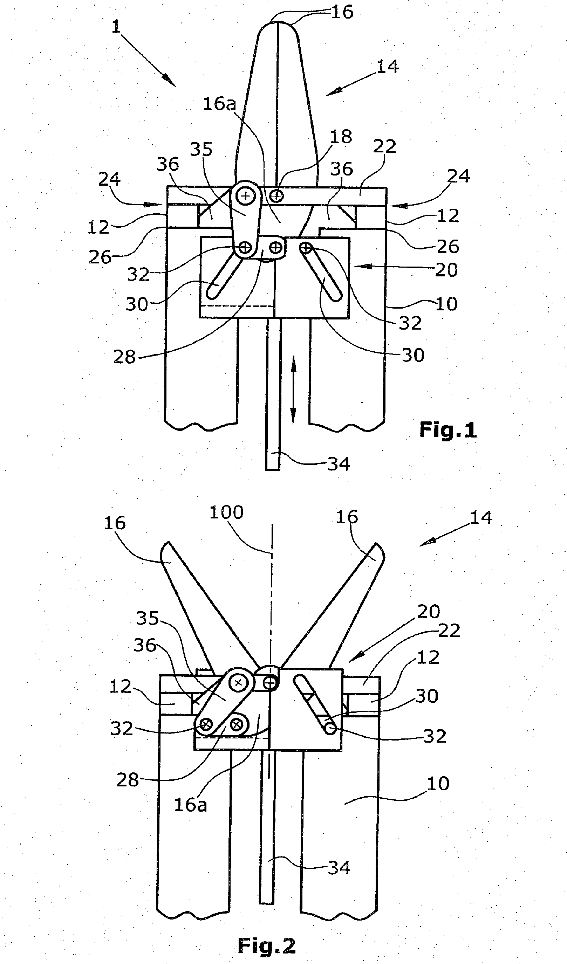

In Fig. 1 und 2 ist ein erstes Ausführungsbeispiel einer erfindungsgemäßen Roboterstruktur 1 schematisch in einer Seitenansicht dargestellt.

Die Roboterstruktur 1 besteht aus einem ersten Roboterelement 10 und einem dazu relativ bewegbaren zweiten Roboterelement 14, welches zwei gleichmäßig gegeneinander bewegbarer Greif- und/oder Schneidelemente 16 aufweist. In Fig. 1 ist dabei die Roboterstruktur 1 mit einer geschlossenen Stellung der Greif- und/oder Schneidelemente 16 und in Fig. 2 mit einer offenen Stellung der Greif- und/oder Schneidelemente 16 gezeigt.

Das zweite Roboterelement 14 ist mit dem ersten Roboterelement 10 verbunden, indem die Greif- und/oder Schneidelemente 16 über eine Gelenkachse 18 in einem Trägerelement 22 gelagert sind, wobei das Trägerelement 22 mit dem ersten Roboterelement 10 verbunden ist.

Zwischen dem Trägerelement 22 und dem ersten Roboterelement 10 sind zwei Sensorelemente 12 angeordnet, wobei das Trägerelement 22 jeweils mit einem sensitiven Ende 24 der Sensorelemente 12 verbunden ist und das erste Roboterelement mit einem Basiselement 26 der Sensorelemente 12 verbunden ist.

Die Sensorelemente 12 messen die durch Greif- und/oder Kontaktkräfte an den Greif- und/oder Schneidelementen 16 verursachten Kräfte und/oder Momente, die über die Gelenkachse 18 und das Trägerelement 22 an das sensitive Ende 24 der Sensorelemente 12 übertragen werden.

Die Roboterstruktur 1 weist eine Kraftübertragungseinrichtung 20 auf, die die Greif- und/oder Schneidelemente 16 antreibt. Dafür weist die Kraftübertragungseinrichtung 20 jeweils zwei identische Antriebsmechanismen auf, die jeweils eines der Greif- und/oder Schneidelemente 16 antreibt.

Im Folgenden wird nur der Antriebsmechanismus für das vom Betrachter aus gesehene rechte Greif- und/oder Schneidelement 16 beschrieben. Der Antriebsmechanismus des linken Greif- und/oder Schneidelementes 16 ist für den Betrachter verdeckt auf der vom Betrachter abgewandten Seite der Kraftübertragungseinrichtung 20 und um 180 ° um die Mittelachse 100 der Roboterstruktur 1 gedreht angeordnet.

Der Antriebsmechanismus besteht aus einem Antriebselement 28, das mit dem unteren Ende 16a des rechten Greif- und/oder Schneidelementes 16 verbunden ist. Zum Erzeugen einer Antriebskraft, die über das Antriebselement 28 auf das Greif- und/oder Schneidelement ausgeübt wird, weist die Kraftübertragungseinrichtung 20, eine Führungskulisse in Form einer Führungsnut 30 auf, in der ein Stift 32 des Antriebselementes 28 geführt ist. Die Führungsnut 30 ist in dem in Fig. 1 und 2 dargestellten Ausführungsbeispiel in einem Winkel zu der Mittelachse 100 der Roboterstruktur angeordnet, wobei die Führungsnut 30 in Richtung des bewegbaren Roboterelementes 14 geneigt ist.

Die Kraftübertragungseinrichtung 20 ist in den Fign. 1 und 2 dargestellten Ausführungsbeispielen über eine Zug/Druckstange 34 antreibbar, wie in Fig. 1 durch den Doppelfeil angedeutet ist. Wird über einen Druck über die Zug/Druckstange 34 die Kraftübertragungseinrichtung 20 in Richtung des bewegbaren Roboterelementes 14 die Kraftübertragungseinrichtung 20 in Richtung zu dem bewegbaren Roboterelement 14 bewegt, erfolgt eine Bewegung des Antriebselementes 28 in eine Richtung orthogonal zu der Mittelachse 100 der Roboterstruktur 1, da der Stift 32 des Antriebselementes 28 durch die Führungsnut 30 von der Mittelachse 100 wegbewegt wird. Dadurch übt das Antriebselement 28 eine Zugkraft auf das untere Ende 16a des rechten Greif- und/oder Schneidelementes 16 aus, sodass das Greif- und/oder Schneidelement 16 in die geöffnete Stellung gezogen wird. Dabei bewirkt ein Führungselement 35 in Form eines Führungshebels, dass während des gesamten Bewegungsvorganges das Antriebselement 28 orthogonal zu der Mittelachse 100 ausgerichtet verbleibt. Dadurch werden von den Antriebskräften nur Kraftkomponenten auf das Greif- und/oder Schneidelement übertragen, die einer Richtung orthogonal zu der Mittelachse 100 verlaufen. Diese Kräfte werden von dem unteren Ende 16a des Greif- und/oder Schneidelementes auf die Gelenkachse 18 und somit auf das Trägerelement 22 übertragen. Dadurch, dass der Antriebsmechanismus für das linke Greif- und/oder Schneidelement 16 um 180 ° gedreht ist, verlaufen die Antriebselemente 28 für die beiden Greif- und/oder Schneidelemente 16 in entgegengesetzte Richtungen, sodass auch die von den Antriebselementen 28 auf die Greif- und/oder Schneidelemente ausgeübten Kräfte einander entgegengerichtet sind. Die entsprechenden Antriebskräfte werden somit zwar auf das Trägerelement 22 über die Gelenkachse 18 übertragen, heben sich jedoch aufgrund ihrer entgegengesetzten Richtung auf. Dadurch werden die Antriebskräfte nicht an das sensitive Ende 24 der Sensorelemente 12 übertragen, sodass diese nicht bei der Messung der Greif- und/oder Kontaktkräfte mitgemessen werden.

Um zu verhindern, dass Kraftkomponenten in axiale Richtung, die beispielsweise durch Reibung der Stifte 32 in den Führungsnuten 30 entstehen können, auf die Antriebselemente 28 und somit auf das Trägerelement 22 übertragen werden, sind die Führungselemente 35 über Verbindungselemente 36 mit dem ersten Roboterelement 10 und/oder mit den Basiselementen 26 der Sensorelemente 12 verbunden. Die Führungselemente 35 und die Verbindungselemente 36 nehmen die Kraftkomponente in axiale Richtung auf und leiten diese an das erste Roboterelement 10 bzw. die Basiselemente 26.

Dadurch wird verhindert, dass von der Kraftübertragungseinrichtung 20 erzeugte Kraftkomponenten in axialer Richtung von den Sensorelementen 12 erfasst werden und in die Messung der Greif und/oder Kontaktkräfte einfließen.

Beim Greif- und oder Schneidvorgang mit Hilfe der erfindungsgemäßen Roboterstruktur 1 wird von dem gegriffenen oder bearbeiteten Objekt auf die Greif- und/oder Schneidelemente 16 die Greif- und/oder Kontaktkraft ausgeübt, wobei die Greif- und/oder Schneidelemente 16 die Kraft als Kräfte und/oder Momente auf die Gelenkachse 18 übertragen. Von der Gelenkachse 18 werden diese Kräfte und/oder Momente über das Trägerelement 22 auf die sensitiven Enden 24 der Sensorelemente 12 übertragen, sodass die Sensorelemente 12 die Kräfte und/oder Momente erfassen können. Über die erhaltenen Werte können dann die tatsächlich vorhandenen Greif- und/oder Kontaktkräfte errechnet werden.

Aufgrund der einfachen mechanischen Ausgestaltung der erfindungsgemäßen Roboterstruktur 1 und nur sehr weniger bewegter Teile ist einer Sterilisation der Roboterstruktur 1 besonders gut möglich, sodass eine derartige Roboterstruktur besonders vorteilhaft für die minimalinvasive Chirurgie ist.

In dem in Fig. 1 und 2 dargestellten Ausführungsbeispiel sind die Antriebselemente 28 als Stange und das Führungselement 35 als Führungshebel ausgeführt. Selbstverständlich ist es auch möglich, dass das Antriebselement 28 beispielsweise über eine separate Führungskulisse derart geführt wird, dass das Antriebselement 28 in der Anordnung orthogonal zu der Mittelachse 100 verbleibt. Die separate Führungskulisse ist dabei vorzugsweise in dem Verbindungselement angeordnet.

Die Führungsfunktion kann beispielsweise auch durch die bereits vorhandene Führungskulisse in Form der Führungsnut 30 erfolgen, indem beispielsweise der Stift 32 derart ausgestaltet ist, dass eine Drehung in der Führungsnut 30 verhindert wird.

Die Kraftübertragungseinrichtung 20 kann an Stelle eines Zugdruckstabs 34 auch über einen federvorgespannten Zug oder einen federvorgespannten Druckstab angetrieben werden. Auch ist es möglich, dass die Kraftübertragungseinrichtung 20 über ein federvorgespanntes Zugseil angetrieben ist.

Bei dem in Fig. 1 und 2 dargestellten Ausführungsbeispiel werden die Greif- und/oder Schneidelemente 16 geöffnet, indem die Kraftübertragungseinrichtung 20 in Richtung des bewegbaren Roboterelementes 14 geschoben wird. Selbstverständlich ist es auch möglich, dass durch einen Zug der Kraftübertragungseinrichtung 20 in entgegengesetzter Richtung ein Öffnen der Greif- und/oder Schneidelemente 16 bewirkt, indem die Führungsnuten 30 in entgegengesetzte Richtung geneigt sind.

Die Kraftübertragung von der Kraftübertragungseinrichtung 20 auf die Antriebselemente 28 muss nicht notwendiger Weise über eine Führungskulisse erfolgen. Selbstverständlich ist es auch möglich, dass eine Kraftübertragung auf andere Art und Weise erfolgt, dass die Antriebselemente 28 während des gesamten Bewegungsvorgangs orthogonal zu der Mittelachse 100 angeordnet sind.

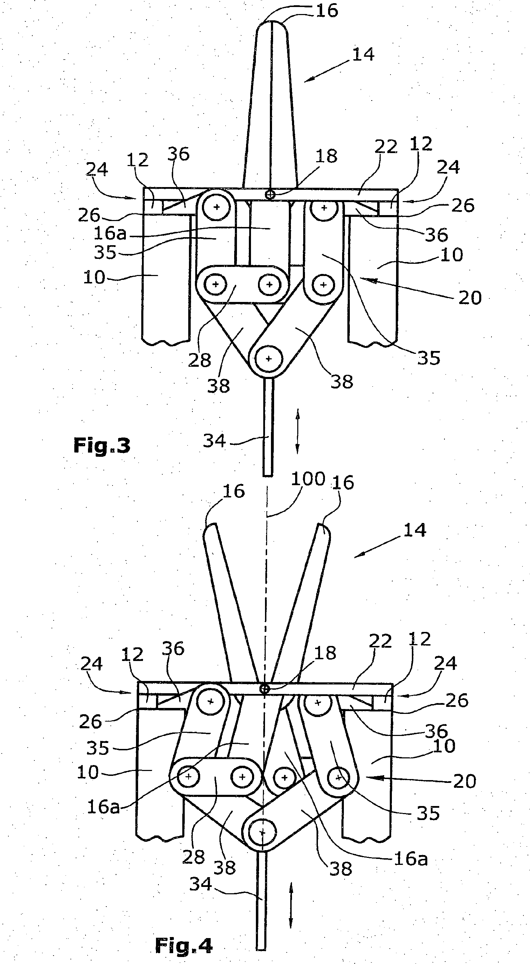

In Fig. 3 und 4 ist eine zweite Ausführungsform einer erfindungsgemäßen Roboterstruktur gezeigt, wobei in Fig. 3 die Roboterstruktur 1 mit geschlossenen Greif- und/oder Schneidelementen 16 und in Fig. 4 mit geöffneten Greif- und/oder Schneidelementen 16 dargestellt ist.

Die in Fig. 3 und 4 dargestellte Roboterstruktur 1 weist im Wesentlichen die gleichen funktionsrelevanten Teile wie das erste Ausführungsbeispiel, das in Fig. 1 und 2 dargestellt ist, auf.

Die Roboterstruktur 1 besteht ebenfalls aus einem ersten Roboterelement 10 und einem relativ dazu bewegbaren zweitem Roboterelement 14, das zwei gleichmäßig gegeneinander bewegbare Greif- und/oder Schneidelemente 16 aufweist. Die Greif- und/oder Schneidelemente 16 sind über eine Gelenkachse 18 in einem Trägerelement 22 gelagert, das mit dem ersten Roboterelement 10 verbunden ist. Dabei sind Sensorelemente 12 zwischen dem Trägerelement 22 und dem ersten Roboterelement 10 angeordnet, sodass auf das Trägerelement 22 ausgeübte Kräfte von den Sensorelementen 12 gemessen werden können, indem das sensitive Ende 24 der Sensorelemente 12 mit dem Trägerelement 22 verbunden sind.

Die Sensorelemente 12 messen die durch Greif- und/oder Kontaktkräfte an dem Greif- und/oder Schneidelementen 16 verursachten Kräfte und/oder Momente.

Zum Bewegen der Greif- und/oder Schneidelemente 16 weist die Roboterstruktur 1 eine Kraftübertragungseinrichtung 22 auf, die über eine Zug/Druckstange 34 antreibbar ist. In dem in Fig. 3 und 4 dargestellten Ausführungsbeispiel weist die Kraftübertragungseinrichtung 20 zwei Hebel 38 auf, die jeweils über Antriebselemente 28 mit den unteren Enden des rechten und des linken Greif- und/oder Schneidelementes 16 verbunden sind. Bei Bewegung der Zugstange 34 schwenken die Hebel 38 auseinander, sodass die unteren Enden 16a der Greif- und/oder Schneidelemente 16 von den Antriebselementen 28 auseinander gezogen werden. Dabei sind die H ebel 38 derart ausgestaltet, dass während des gesamten Bewegungsvorganges die Antriebselemente 28 orthogonal zu der Mittelachse 100 ausgerichtet sind. Dadurch werden von den Antriebskräften nur Kraftkomponenten auf die Greif- und/oder Schneidelemente 16 übertragen, die in eine Richtung orthogonal zu der Mittelachse 100 verlaufen. Diese Kräfte werden von dem unteren Ende 16a der Greif- und/oder Schneidelemente auf die Gelenkachse 18 und somit auf das Trägerelement 22 übertragen. Dadurch, dass bei dem in Fig. 3 und 4 dargestellten Ausführungsbeispiel der Antriebsmechanismus für das linke Greif- und/oder Schneidelement 16 im Vergleich zu dem Antriebsmechanismus für das rechte Greif- und/oder Schneidelement 16 um 180 ° gedreht angeordnet ist, werden auf das Trägerelement 22 Kräfte in entgegengesetzte Richtung übertragen, die sich gegeneinander aufheben. Dadurch werden die Antriebskräfte nicht in das sensitive Ende 24 der Sensorelemente 12 übertragen.

Um zu verhindern, dass Kraftkomponenten in axiale Richtung der Roboterstruktur 1 auf die Antriebselemente und somit auf das Trägerelement 22 übertragen werden, sind Führungselemente 35 über Verbindungselemente 36 mit dem ersten Roboterelement 10 und/oder mit den Basiselementen 26 der Sensorelemente 12 verbunden. Die entsprechenden axialen Kraftkomponenten werden von den Führungselementen 35 auf das erste Roboterelement 10 und/oder die Basiselemente 26 der Sensorelemente 12 übertragen. Dadurch kann verhindert werden, dass von der Kraftübertragungseinrichtung 20 erzeugte Kraftkomponenten in axiale Richtung von den Sensorelementen 12 erfasst werden und in die Messung der Greif- und/oder Kontaktkräfte einfließen.

## Patentansprüche

1. Roboterstruktur (1), insbesondere für die minimal-invasive Chirurgie, mit
einem ersten Roboterelement (10) und einem dazu relativ bewegbaren zweiten Roboterelement (14), welches zwei gleichmäßig gegeneinander bewegbare Greif- und/oder Schneideelemente (16) aufweist, die über eine Gelenkachse (18) mit dem ersten Roboterelement (10) verbunden sind,
einer Kraftübertragungseinrichtung (20) zum Bewegen der Greif- und/oder Schneidelemente (16) des bewegbaren Roboterelements (14) und
mindestens einem Sensorelement (12) mit einem sensitiven Ende (24) zur Aufnahme von an dem bewegbaren Roboterelement (14) auftretenden Kräften und/oder Momenten und mit einem fest mit dem ersten Roboterelement (10) verbundenes Basiselement (26) aufweist,
**dadurch gekennzeichnet, dass**
zur Aufnahme der durch Greif- und/oder Kontaktkräfte an den Greif- und/oder Schneidelementen (16) verursachten Kräfte und/oder Momente die Gelenkachse (18) in einem Trägerelement (22) gelagert und das Trägerelement (22) mit dem sensitiven Ende (24) jedes Sensorelements (12) verbunden ist, und
dass die Kraftübertragungseinrichtung (20) jeweils über ein Antriebselement (28) mit einem der Greif- und/oder Schneidelemente (16) zum Einleiten der Bewegung verbunden ist, wobei die Antriebselemente (28) einander entgegengerichtet und im Wesentlichen orthogonal zu der Mittelachse (100) der Roboterstruktur (1) angeordnet sind und während des gesamten Bewegungsvorgangs der Greif- und/oder Schneidelemente (16) in der orthogonalen Anordnung zu der Mittelachse (100) verbleiben.

2. Roboterstruktur nach Anspruch 1, **dadurch gekennzeichnet, dass** jedes Antriebselement (28) als Stange ausgebildet ist.

3. Roboterstruktur nach Anspruch 1 oder 2, **dadurch gekennzeichnet, dass** jeweils ein Führungselemente (35) ein Antriebselement (28) führt, derart, dass die Antriebselemente (28) während des gesamten Bewegungsvorgangs der Greif- und/oder Schneidelemente (16) in der orthogonalen Anordnung zu der Mittelachse (100) verbleiben.

4. Roboterstruktur nach Anspruch 3, **dadurch gekennzeichnet, dass** die Führungselemente (35) jeweils als Führungshebel ausgebildet sind.

5. Roboterstruktur nach Anspruch 3 oder 4, **dadurch gekennzeichnet, dass** die Führungselemente (35) jeweils mit dem ersten Roboterelement (10) und/oder mit dem Basiselement (26) eines der Sensorelemente (12) verbunden sind, vorzugsweise über ein Verbindungselement (36).

6. Roboterstruktur nach einem der Ansprüche 1 bis 5, **dadurch gekennzeichnet, dass** die Kraftübertragungseinrichtung (20) die Antriebselemente (28) jeweils über eine Kulissenführung antreibt, wobei die Antriebselemente vorzugsweise Stifte (32) aufweisen, die in die Kulissenführung führbar sind.

7. Roboterstruktur nach Anspruch 6, **dadurch gekennzeichnet, dass** die Kulissenführungen jeweils eine Führungsnut (30) aufweisen, die in einem Winkel zu der Mittelachse (100) der Roboterstruktur (1) angeordnet ist.

8. Roboterstruktur nach einem der Ansprüche 1 bis 5, **dadurch gekennzeichnet, dass** die Kraftübertragungseinrichtung (20) die Antriebselemente (28) jeweils über einen Hebel (38) antreibt.

9. Roboterstruktur nach einem der Ansprüche 1 bis 8, **dadurch gekennzeichnet, dass** die Kraftübertragungseinrichtung (20) über eine Zug/Druckstange (34) antreibbar ist.

10. Roboterstruktur nach einem der Ansprüche 1 bis 8, **dadurch gekennzeichnet, dass** die Kraftübertragungseinrichtung (20) über eine Zugstange oder über eine Druckstange antreibbar ist, wobei die Zugstange oder die Druckstange vorzugsweise federvorgespannt ist.

11. Roboterstruktur nach einem der Ansprüche 1 bis 8, **dadurch gekennzeichnet, dass** die Kraftübertragungseinrichtung (20) über eine Zugseil antreibbar ist, wobei das Zugseil vorzugsweise federvorgespannt ist.

## Claims

1. A robot structure (1), in particular for minimally invasive surgery, comprising:
a first robot element (10) and a second robot element (14) that can be moved relative to the first robot element, said second robot element having two grasping and/or cutting elements (16) which can be uniformly moved with respect to each other and which are connected to the first robot element (10) via a hinge axis (18),
a force transmitting device (20) for moving the grasping and/or cutting elements (16) of the movable robot element (14) and
at least one sensor element (12) comprising a sensitive end (24) for receiving forces and/or moments that occur on the movable robot element (14) and comprising a base element (26) that is fixed to the first robot element (10),
**characterized in that**
the hinge axis (18) is supported in a support element (22) in order to receive the forces and/or moments that are caused by grasping and/or contact forces on the grasping and/or cutting elements (16), and the support element (22) is connected to the sensitive end (24) of each sensor element (12), and
the force-transmitting device (20) is connected to one of the grasping and/or cutting elements (16) via a driving element (28), respectively, in order to initiate the movement, with the driving elements (28) being disposed opposed to each other and in a substantially orthogonal manner with respect to the central axis (100) of the robot structure (1) and maintaining the orthogonal orientation with respect to the central axis (100) during the entire moving process of the grasping and/or cutting elements (16).

2. The robot structure of claim 1, **characterized in that** each driving element (28) is designed as a rod.

3. The robot structure of claim 1 or 2, **characterized in that** a respective guiding element (35) guides a driving element (28) such that the driving elements (28) maintain the orthogonal orientation with respect to the central axis (100) during the entire moving process of the grasping and/or cutting elements (16).

4. The robot structure of claim 3, **characterized in that** the guiding elements (35) are each designed as guiding levers.

5. The robot structure of claims 3 and 4, **characterized in that** the guiding elements (35) are each connected with the first robot element (10) and/or the base element (26) of one of the sensor elements (12), preferably via a connecting element (36).

6. The robot structure of one of claims 1 to 5, **characterized in that** the force transmitting device (20) drives the driving elements (28), respectively, via a slotted guide device, the driving elements preferably comprising pins (32) adapted to be guided in the slotted guide device.

7. The robot structure of claim 6, **characterized in that** the slotted guide devices each comprise a guiding groove (30) arranged under an angle with respect to the central axis (100) of the robot structure (1).

8. The robot structure of one of claims 1 to 5, **characterized in that** the force transmitting device (20) drives the driving elements (28) via a lever (38), respectively.

9. The robot structure of one of claims 1 to 8, **characterized in that** the force transmitting device (20) is adapted to be driven via a pull/push rod (34).

10. The robot structure of one of claims 1 to 8, **characterized in that** the force transmitting device (20) is adapted to be driven via a pull rod or via a push rod, the pull rod or the push rode preferably being spring-biased.

11. The robot structure of one of claims 1 to 8, **characterized in that** the force transmitting device (20) is adapted to be driven via a pull cable, the pull cable preferably being spring-biased.

## Revendications

1. Structure de robot (1), en particulier pour la chirurgie faiblement invasive, avec
un premier élément de robot (10) et un deuxième élément de robot (14) mobile par rapport au premier et qui présente deux éléments de préhension et/ou de découpe (16) mobiles uniformément l'un par rapport à l'autre et qui sont raccordés au premier élément de robot (10) par le biais d'un axe articulé (18),
un dispositif de transmission de force (20) pour le déplacement des éléments de préhension et/ou de découpe (16) de l'élément de robot (14) mobile, et
au moins un élément de capteur (12) avec une extrémité sensible (24) pour l'enregistrement de forces et/ou de moments apparaissant sur l'élément de robot (14) mobile et avec un élément de base (26) raccordé fixement au premier élément de robots (10),
**caractérisée en ce que**,
pour la reprise des forces et/ou moments provoqués par des forces de saisie et/ou de contact sur les éléments de préhension et/ou de découpe (16), l'axe articulé (18) est supporté dans un élément de support (22), et l'élément de support (22) est raccordé à l'extrémité sensible (24) de chaque élément de capteur (12), et
**en ce que** le dispositif de transmission de force (20) est raccordé respectivement par le biais d'un élément d'entraînement (28) à un des éléments de préhension et/ou de découpe (16) pour la mise en oeuvre du mouvement, les éléments d'entraînement (28) étant dirigés de façon opposée les uns aux autres et étant disposés de façon essentiellement orthogonale par rapport à l'axe médian (100) de la structure de robot (1) et demeurant dans la disposition orthogonale par rapport à l'axe médian (100) pendant la totalité du processus de mouvement des éléments de préhension et/ou de découpe (16).

2. Structure de robot selon la revendication 1, **caractérisée en ce que** chaque élément d'entraînement (28) est constitué en tant que tige.

3. Structure de robot selon la revendication 1 ou 2, **caractérisée en ce que** respectivement un élément de guidage (35) guide un élément d'entraînement (28) de telle sorte que les éléments d'entraînement (28) demeurent dans la disposition orthogonale par rapport à l'axe médian (100) pendant la totalité du processus de mouvement des éléments de préhension et/ou de découpe (16).

4. Structure de robot selon la revendication 3, **caractérisée en ce que** les éléments de guidage (35) sont respectivement constitués en tant que leviers de guidage.

5. Structure de robot selon la revendication 3 ou 4, **caractérisée en ce que** les éléments de guidage (35) sont raccordés respectivement au premier élément de robot (10) et/ou à l'élément de base (26) d'un des éléments de capteur (12), de préférence par le biais d'un élément de raccordement (36).

6. Structure de robot selon une des revendications 1 à 5, **caractérisée en ce que** le dispositif de transmission de force (20) entraîne les éléments d'entraînement (28) respectivement par le biais d'un guide à coulisse, les éléments d'entraînement présentant de préférence des broches (32) qui peuvent être guidées dans le guide à coulisse.

7. Structure de robot selon la revendication 6, **caractérisée en ce que** les guides à coulisse présentent respectivement une rainure de guidage (30) qui est disposée en formant un angle par rapport à l'axe médian (100) de la structure de robot (1).

8. Structure de robot selon une des revendications 1 à 5, **caractérisée en ce que** le dispositif de transmission de force (20) entraîne les éléments d'entraînement (28) respectivement par le biais d'un levier (38).

9. Structure de robot selon une des revendications 1 à 8, **caractérisée en ce que** le dispositif de transmission de force (20) peut être entraîné par le biais d'une tige de traction/pression (34).

10. Structure de robot selon une des revendications 1 à 8, **caractérisée en ce que** le dispositif de transmission de force (20) peut être entraîné par le biais d'une tige de traction ou par le biais d'une tige de pression, la tige de traction ou la tige de pression étant de préférence pré-tendue par ressort.

11. Structure de robot selon une des revendications 1 à 8, **caractérisée en ce que** le dispositif de transmission de force (20) peut être entraîné par le biais d'un câble de traction, le câble de traction est de préférence pré-tendu par ressort.
